# EUROPÄISCHE PATENTSCHRIFT

(11) **EP 0 751 143 B1**
(45) Veröffentlichungstag und Bekanntmachung des Hinweises auf die Patenterteilung: **05.09.2001**
(21) Anmeldenummer: 96109834.0
(22) Anmeldetag: 19.06.1996
(51) Int. Cl.: C07C 2/86, C07C 13/32, C07F 17/00, C07C 13/28

(54) **Verfahren zur Herstellung einer kohlenstoffverbrückten Biscyclopentadienverbindung**
Process for the preparation of carbon bridged dicyclopentadiene compounds
Procédé de préparation de composés de dicyclopentadiène ponté par des atomes de carbone

(30) Priorität: 30.06.1995 DE 19523595
(43) Veröffentlichungstag der Anmeldung: 02.01.1997
(73) Patentinhaber: Basell Polyolefine GmbH, 77694 Kehl (DE)
(72) Erfinder: Küber, Frank, Dr., 61440 Oberursel (DE); Riedel, Michael, Dr., 60529 Frankfurt (DE)

(56) Entgegenhaltungen:
- EP-A- 0 722 949
- US-A- 5 030 757
- E. GHERA ET AL: J. AM. CHEM. SOC., Bd. 82, 1960, Seiten 4945-4952, XP002057983

## Beschreibung

Die vorliegende Erfindung bezieht sich auf ein Verfahren zur Herstellung einer kohlenstoffverbrückten Biscyclopentadienverbindung und die Verwendung dieses Verfahrens als Teilschritt bei der Herstellung eines kohlenstoffverbrückten Biscyclopentadienyl-Metallocens, welches als Katalysatorkomponente, z.B. für die Herstellung von Polyolefinen, eingesetzt werden kann.

Aus der Literatur ist die Herstellung von Polyolefinen in Gegenwart von Metallocenen in Kombination mit Aluminoxanen oder anderen Cokatalysatoren, die aufgrund ihrer Lewis-Acidität das neutrale Metallocen in ein Kation überführen und stabilisieren können, bekannt.

Metallocene und Halbsandwichkomplexe sind nicht nur hinsichtlich der Polymerisation oder Oligomerisation von Olefinen von großem Interesse, sie können auch als Hydrier-, Epoxidations-, Isomerisierungs-, C-C-Kupplungskatalysatoren eingesetzt werden (Chem. Rev. 1992, 92, 965-994).

Kohlenstoffverbrückte Metallocene sind in der Literatur beschrieben (US 4,892,851; EP 416 566). Die Synthese dieser Metallocene verläuft über die Darstellung des kohlenstoffverbrückten Biscyclopentadienligandsystems, welche in mehreren Stufen durchgeführt werden muß und nur mit sehr geringen Ausbeuten verläuft.

Aus EP 456 455 ist bekannt, quartäre Ammoniumverbindungen bei der Alkylierung von Cyclopentadienen zu verwenden.

Aus Organometallics, 10, 1991, Seite 3739-3745 ist bekannt, Triethylbenzylammoniumchlorid in der Synthese von Biscyclopentadienyldimethylmethan einzusetzen.

Aus der Literatur ist bekannt, daß Cyclopentadien mit cyclischen Ketonen, unter Zusatz einer Base, direkt zu verbrücktem Biscyclopentadienliganden umgesetzt werden kann (J. Chem. Research (S), 1992, 162). Diese Synthese verläuft mit niedrigen Ausbeuten und bedarf anschließend einer aufwendigen chromatographischen Reinigung.

Es bestand somit die Aufgabe ein Herstellungsverfahren für kohlenstoffverbrückte Biscyclopentadienverbindungen zur Verfügung zu stellen, welches die Nachteile des Standes der Technik vermeidet.

Die vorliegende Erfindung betrifft somit ein Verfahren zur Herstellung einer kohlenstoffverbrückten Biscyclopentadienverbindung durch Umsetzung einer oder zweier Cyclopentadienverbindungen LH, wovon mindestens eine Cyclopentadienverbindung eine substituierte Cyclopentadienverbindung ist, mit einer Carbonylverbindung in Gegenwart mindestens einer Base und mindestens eines Phasentransferkatalysators, wobei das Verfahren in einem Zweiphasensystem durch geführt wird, welches aus einem organischen Lösemittel und Wasser besteht.

Die kohlenstoffverbrückte Biscyclopentadienverbindung weist vorzugsweise die Formel I auf 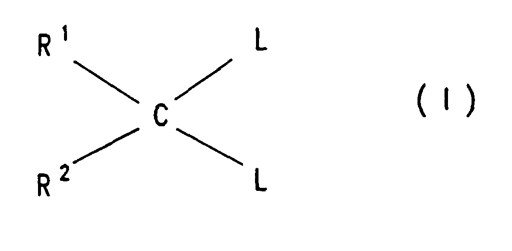 , worin L unabhängig voneinander gleich oder verschieden eine Cyclopentadiengruppe sind, wobei mindestens eine Gruppe L eine substituierte Cyclopentadienylgruppe ist, und R¹ und R² gleich oder verschieden sind und ein Wasserstoffatom oder einen C₁-C₃₀-Kohlenwasserstoffrest bedeuten.

Die Cyclopentadiengruppen L in Formel I können unsubstituiert oder substituiert sein. Sie sind gleich oder verschieden, bevorzugt gleich.

Beispiele für substituierte Cyclopentadiengruppen L sind: Tetramethylcyclopentadien, 3-Methylcyclopentadien, 3-tert.butylcyclopentadien, Methyl-tert.butylcyclopentadien, Isopropylcyclopentadien, Dimethylcyclopentadien, Trimethylcyclopentadien, Trimethylethylcyclopentadien, 3-Phenylcyclopentadien, Diphenylcyclopentadien, Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenaphthinden, 2-Methyl-4,6-diisopropylinden, Fluoren, 2-Methylfluoren oder 2,7-Di-tert.butylfluoren.

Eine oder beide Cyclopentadiengruppen L sind eine substituierte Cyclopentadiengruppe, insbesondere ein Indenderivat wie Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenanaphthinden, 2-Methyl-4,6-diisopropylinden oder ein Fluorenylderivat wie Fluoren, 2-Methylfluoren oder 2,7-Di-tert.butylfluoren.

Die Reste R¹ und R² sind gleich oder verschieden, bevorzugt gleich, und bedeuten C₁-C₃₀-Kohlenwasserstoffreste wie C₁-C₁₀-Alkyl oder C₆-C₁₄-Aryl. Die Reste R¹ und R² können auch zusammen mit den sie verbindenen Atomen ein Ringsystem bilden, welches bevorzugt 4 bis 40, besonders bevorzugt 5 bis 15 Kohlenstoffatome enthält.

Beispiele für kohlenstoffverbrückte Biscyclopentadienverbindungen der Formel I sind:
2,2-(Bisinden)propan, 2,2-(Bisinden)butan, 2,2-(Bisinden)methan, 2,2-(Bisinden)cyclopentan, 2,2-(Bisinden)cyclohexan, 1,1-(Bisinden)-1-phenyl-ethan, 1,1-(Bisinden)ethan, 1,1-(Bisinden)propan, 2,2-(Bis(2'-methyl-4'-phenylinden))propan, 2,2-(Bis(2 '-ethyl-4'-phenylinden))propan, 2,2-(Bis(2'-methyl-4'-naphthylinden))propan, 2,2-(Bis(2'-methyl-4',5'-benzoinden))propan, 1,1-(Bis(2'-methyl-4'-phenylinden))-1-phenyl-ethan, 1,1-(Bis(2'-ethyl-4'-phenylinden))-1-phenyl-ethan, 1,1-(Bis(2'-methyl-4'-naphthyl-inden))-1-phenyl-ethan, 2,2-(Biscyclopentadien)butan, 2,2-(Bis(methylcyclopentadien))propan, (2-Cyclopentadien-2-fluoren)propan, (2-(3'-Methylcyclopentadien)-2-fluoren)propan, (2-Inden-2-fluoren)propan, (2-Cyclopentadien-2-inden)propan, (1-Cyclopentadien-1-fluoren)-1-phenyl-ethan, (1-Inden-1-fluoren)-1-phenyl-ethan, (2-(3'-tert.butylcyclopentadienyl)-2-fluoren)propan, (1-Cyclopentadien-1-inden)-1-phenyl-ethan.

Zur Herstellung von Biscyclopentadienverbindungen der Formel I, in denen die beiden Cyclopentadiengruppen L gleich sind, wird eine Cyclopentadienverbindung LH eingesetzt. Zur Herstellung von Biscyclopentadienverbindungen der Formel I, in denen die beiden Cyclopentadiengruppen L verschieden sind, werden zwei voneinander verschiedene Cyclopentadienverbindungen LH eingesetzt.

Die in dem erfindungsgemäßen Verfahren eingesetzten Cyclopentadienverbindungen LH können substituiert oder unsubstituiert sein, wobei mindestens eine Cyclopentadienverbindung eine substituierte Cyclopentadienverbindung ist.

Beispiele für substituierte Cyclopentadienverbindungen LH sind Tetramethylcyclopentadien, Methylcyclopentadien, tert.butylcyclopentadien, Methyl-tert.butylcyclopentadien, Isopropylcyclopentadien, Dimethylcyclopentadien, Trimethylcyclopentadien, Trimethylethylcyclopentadien, Phenylcyclopentadien, Diphenylcyclopentadien, Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenaphthinden, 2-Methyl-4,6-diisopropylinden, Fluoren, 2-Methylfluoren oder 2,7-Di-tert.butylfluoren.

Eine oder beide der in dem erfindungsgemäßen Verfahren eingesetzten Cyclopentadienverbindungen LH sind eine substituierte Cyclopentadienverbindung, insbesondere ein Indenderivat wie Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenanaphthinden, 2-Methyl-4,6-diisopropylinden oder ein Fluorenylderivat wie Fluoren, 2-Methylfluoren oder 2,7-Di-tert.butylfluoren.

Die in dem erfindungsgemäßen Verfahren eingesetzten Carbonylverbindungen sind vorzugsweise Ketone wie Aceton, Acetophenon, Benzophenon, Cyclohexanon, Cyclopentanon, 2-Hexanon, 2-Butanon, 2-Methyl-3-pentanon oder 2,2-Dimethyl-3-butanon oder Aldehyde wie Acetaldehyd oder Benzaldehyd.

Als Base können Hydroxide der Elemente der Gruppe la, IIa oder IIIa des Periodensystems der Elemente wie LiOH, NaOH, KOH, RbOH, Mg(OH)₂, Ca(OH)₂ und Sr(OH)₂ eingesetzt werden. Bevorzugt wird eine Base eingesetzt, z.B. LiOH, NaOH oder KOH.

Als Phasentransferkatalysator können quartäre Ammoniumsalze und Phosphoniumsalze der allgemeinen Form [R³₄Z]⁺X⁻ eingesetzt werden, wobei R³ gleich oder verschieden sind und ein Wasserstoffatom, ein Halogenatom, oder eine C₁-C₄₀-kohlenstoffhaltige Gruppe wie eine C₁-C₂₀-Alkyl-, eine C₁-C₁₀-Alkoxy-, eine C₆-C₂₀-Aryl-, eine C₂-C₁₂-Alkenyl-, ein C₇-C₄₀-Arylalkyl-, eine C₇-C₄₀-Alkylaryl-, oder eine C₈-C₄₀-Arylalkenylgruppe bedeuten, die jeweils Reste wie -NR⁴₃, -SR⁴₂, -SiR⁴₃ oder -OSiR⁴₃ tragen können, worin R⁴ gleich oder verschieden ein Halogenatom, eine C₁-C₁₀ Alkylgruppe oder eine C₆-C₁₀ Arylgruppe sind, oder zwei oder mehr Reste R³ zusammen mit den sie verbindenen Atomen ein Ringsystem bilden können, welches bevorzugt 4 bis 40, besonders bevorzugt 5 bis 15 Kohlenstoffatome enthält, Z gleich Stickstoff oder Phosphor bedeutet und X⁻ gleich ein Halogenid, Hydroxid, Tetrahalogenborat, (z.B. Tetrafluoroborat), Hydrogensulfat, Sulfat oder Hexahalogenphosphat, (z.B. Hexafluorophosphat) ist.

Beispiele für als Phasentransferkatalysatoren geeignete Verbindungen sind:
Benzyltrimethylammoniumchlorid,
Benzyltrimethylammoniumhydroxid (insbesondere als wäßrige 40%-ige Lösung), Hexadecyltrimethylammoniumbromid,
Hexadecyltrimethylammoniumchlorid (insbesondere als wäßrige 50%-ige Lösung),
Ethylhexadecyldimethylammoniumbromid,
Tetraethylammoniumtetrafluoroborat,
Tetraethylammoniumbromid,
Tetraethylammoniumhydroxid (insbesondere als wäßrige 20%-ige Lösung),
Benzyltriethylammoniumchlorid,
Benzyltriethylammoniumhydroxid,
Tetrapropylammoniumbromid,
Tetrabutylammoniumchlorid,
Tetrabutylammoniumfluorid-trihydrat,
Tetrabutylammoniumtetrafluoroborat,
Tetrabutylammoniumhydrogensulfat,
Tetrabutylammoniumhydroxid (insbesondere als 12,5%-ige Lösung in Methanol)
Benzeltributylammoniumbromid,
Tetraoctylammoniumbromid,
Methyltrioctylammoniumchlorid,
Tetrabutylphosphoniumbromid,
Tetrabutylphosphoniumchlorid,
Tributylhexadecylphosphoniumbromid,
Ethyltrioctylphosphoniumbromid,
Butyltriphenylphosphoniumchlorid
und Tetraphenylphosphoniumbromid.

Weiterhin können als Phasentransferkatalysatoren Kronenverbindungen, insbesondere solche der allgemeinen Formel III eingesetzt werden, 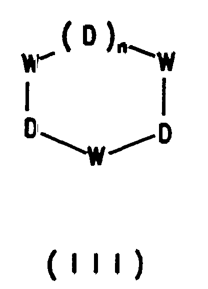 worin D gleich S, O, NR⁵, PR⁵ ist und R⁵ gleich oder verschieden sind und ein Wasserstoffatom, ein Halogenatom, eine C₁-C₄₀-kohlenstoffhaltige Gruppe wie eine C₁-C₂₀-Alkyl-, eine C₁-C₁₀-Alkoxy-, eine C₆-C₂₀-Aryl-, eine C₂-C₁₂-Alkenyl-, ein C₇-C₄₀-Arylalkyl-, eine C₇-C₄₀-Alkylaryl- oder eine C₈-C₄₀-Arylalkenylgruppe bedeutet, die jeweils Reste -NR⁶₃, -SR⁶₂, -SiR⁶₃ oder -OSiR⁶₃ tragen können, worin R⁶ gleich oder verschieden ein Halogenatom, eine C₁-C₁₀ Alkylgruppe oder eine C₆-C₁₀ Arylgruppe sind, W gleich oder verschieden [R⁷₂C]ₙ ist, wobei R⁷ gleich oder verschieden sind, und ein Wasserstoffatom, ein Halogenatom, eine C₁-C₄₀-kohlenstoffhaltige Gruppe wie eine C₁-C₂₀-Alkyl-, eine C₁-C₁₀-Alkoxy-, eine C₆-C₂₀-Aryl-, eine C₂-C₁₂-Alkenyl-, ein C₇-C₄₀-Arylalkyl-, eine C₇-C₄₀-Alkylaryl-, oder eine C₈-C₄₀-Arylalkenylgruppe bedeutet, die jeweils Reste -NR⁸₃, -SR⁸₂, -SiR⁸₃ oder -OSiR⁸₃ tragen können, worin R⁸ ein Halogenatom, eine C₁-C₁₀ Alkylgruppe oder eine C₆-C₁₀ Arylgruppe ist, oder zwei oder mehr Reste R⁷ zusammen mit den sie verbindenen Atomen ein Ringsystem bilden können, welches bevorzugt 4 bis 40, besonders bevorzugt 5 bis 15 Atome, insbesondere Kohlenstoffatome, enthält, n eine ganze Zahl von 1 bis 40, bevorzugt 1 bis 5 ist.

Beispiele für Kronenverbindungen sind:
12-Krone-4, 15-Krone-5, Benzo-15-Krone-5, 18-Krone-6, Decyl-18-Krone-6, Dibenzo-18-Krone-6, Dicyclohexyl-18-Krone-8, Dibenzo-24-Krone-8, (+)-18-Krone-6-tetracarbonsäure, N-Phenyl-aza-15-Krone-5, ®Kryptofix 21, ® Kryptofix 22, ® Kryptofix 22 DD, ® Kryptofix 23, Tris[2-(-methoxyethoxy)-ethyl]amin, ® Kryptofix 5, ® Kryptofix 111, ® Kryptofix 211, ® Kryptofix 221, ® Kryptofix 221 D, ® Kryptofix 222, ® Kryptofix 222 B (50%-ige Lösung in Toluol), ® Kryptofix 222 BB, ® Kryptofix 222 CC (50%-ige Lösung in Toluol), ® Kryptofix 222 D (50%-ige Lösung in Toluol), ® Kryptofix 221 B (Polymer), und ® Kryptofix 222 B (Polymer).

Bevorzugt wird ein dem erfindungsgemäßen Verfahren ein Phasentransferkatalysator eingesetzt. Die Konzentration des Phasentransferkatalysators kann 0,1 bis 100 Mol-%, bezogen auf die eingesetzte Menge an Cyclopentadienverbindung(en) LH, besonders bevorzugt 1 bis 20 Mol-% betragen.

Das erfindungsgemäße Verfahren wird in einem Zweiphasensystem in Gegenwart mindestens einer Base und mindestens eines Phasentransferkatalysators durchgeführt, wobei eine Phase ein organisches Lösemittel z.B. ein aromatisches Lösemittel wie Toluol, Xylol oder ein aliphatisches Lösemittel wie Tetrahydrofuran, Hexan oder Dichlormethan darstellt und die zweite Phase Wasser ist. Insbesondere sind Zweiphasensysteme Toluol/Wasser, Dichlormethan/Wasser und Tetrahydrofuran/Wasser bevorzugt. Die Konzentration an Base in der wäßrigen Phase kann zwischen 5- und 70 Gew.-% betragen, bevorzugt 25 bis 60 Gew.-%.

Zur Synthese von kohlenstoffverbrückten Biscyclopentadienverbindungen mit zwei gleichen Cyclopentadiengruppen L kann die Cyclopentadienverbindung LH im Überschuß (bezogen auf die Carbonylverbindung) eingesetzt werden, bevorzugt werden 2 bis 3 Äquivalente der Cyclopentadienverbindung LH, bezogen auf die eingesetzte Carbonylverbindung (z.B. Aceton oder Acetophenon) verwendet. Bei der Synthese von kohlenstoffverbrückten Biscyclopentadienverbindungen mit zwei voneinander verschiedenen Cyclopentadiengruppen L, werden zwei voneinander verschiedene Cyclopentadienverbindungen LH eingesetzt. Dabei wird zuerst eine der beiden Cyclopentadienverbindungen mit der Carbonylverbindung zur Reaktion gebracht, wobei das Verhältnis der beiden Komponenten ungefähr 1 : 1 beträgt. Nach einer Reaktionszeit, die zwischen 30 min und 100 h, vorzugsweise zwischen 30 min und 20 h liegen kann, erfolgt die Zugabe der zweiten Cyclopentadienverbindung.

Die Reaktionstemperatur kann zwischen 0°C und 100°C betragen, bevorzugt 0°C bis 30°C. Die Reaktionszeiten liegen in der Regel zwischen 30 min und 100 h, vorzugsweise zwischen 30 min und 20 h.

Das Volumenverhältnis Organische Phase/Wasser (z.B. Toluol/Wasser, Dichlormethan/Wasser oder Tetrahydrofuran/Wasser) kann zwischen 10000:1 und 1:50, bevorzugt zwischen 100:1 und 1:10, besonders bevorzugt zwischen 10:1 und 1:1 liegen.

Bevorzugt wird eine Mischung der Cyclopentadienverbindung LH und der Carbonylverbindung in dem organischen Lösungsmittel vorgelegt und die wäßrige Phase, in der sich sowohl die Base sowie auch der Phasentransferkatalysator befindet, zudosiert. Auch die umgekehrte Reaktionsführung ist möglich. Weiterhin kann zu dem Zweiphasensystem (z.B. Toluol/Wasser, Dichlormethan/Wasser oder Tetrahydrofuran/Wasser), in dem die Cyclopentadienverbindung LH, die Base und der Phasentranfserkatalysator enthalten sind, die Carbonylverbindung über einen Zeitraum von 1 min bis 100 h, bevorzugt von 15 min bis 4 h, zugetropft werden.

Die mit dem erfindungsgemäßen Verfahren erhältlichen kohlenstoffverbrückten Biscyclopentadienverbindungen können als Doppelbindungsisomere anfallen.

Das erfindungsgemäße Verfahren zeichnet sich insbesondere dadurch aus, daß kohlenstoffverbrückte Biscyclopentadienverbindungen in einer einfachen, einstufigen Synthese in hoher Ausbeute erhalten werden können. Das Substitutionsmuster der Verbrückung (R¹R²C) sowie der Cyclopentadiengruppen L kann dabei in einem weiten Bereich variiert werden.

Gegenstand der vorliegenden Erfindung ist ferner die Verwendung des erfindungsgemäßen Verfahrens als Teilschritt eines Verfahrens zur Herstellung eines kohlenstoffverbrückten Biscyclopentadienyl-Metallocens, insbesondere eines kohlenstoffverbrückten Biscyclopentadienyl-Metallocens der nachstehenden Formel V 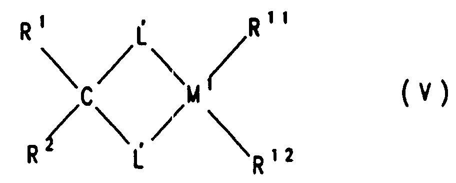
, worin M¹ ein Element der Gruppen IIIb, IVb, Vb oder VIb des Periodensystems der Elemente, insbesondere der Gruppe IVb ist,
worin L' unabhängig voneinander gleich oder verschieden eine Cyclopentadienylgruppe sind, wobei mindestens eine Cyclopentadiengruppe L' eine substituierte Cyclopentadiengruppe ist, R¹ und R² gleich oder verschieden sind, und Wasserstoff oder einen C₁-C₃₀-Kohlenwasserstoffrest wie C₁-C₁₀-Alkyl oder C₆-C₁₄-Aryl bedeuten, die Reste R¹ und R² zusammen mit den sie verbindenen Atomen ein Ringsystem bilden, welches bevorzugt 4 bis 40, besonders bevorzugt 5 bis 15 Kohlenstoffatome enthält, und
R¹¹ und R¹² gleich oder verschieden sind und Wasserstoff, ein Halogenatom oder einen C₁-C₄₀-kohlenstoffhaltigen Rest wie C₁-C₁₀-Alkyl, C₁-C₁₀-Alkoxy, C₆-C₁₄-Aryl, C₆-C₁₄-Aryloxy, C₂-C₁₀-Alkenyl, C₇-C₄₀-Arylalkyl, C₇-C₄₀-Alkylaryl, C₈-C₄₀-Arylalkenyl, Hydroxy, NR⁵₂, worin R⁵ ein C₁-C₁₀-Alkyl, C₁-C₁₀-Alkoxy, C₆-C₁₄-Aryl, C₆-C₁₄-Aryloxy, C₂-C₁₀-Alkenyl, C₇-C₄₀-Arylalkyl, C₇-C₄₀-Alkylaryl oder C₈-C₄₀-Arylalkenyl bedeuten.

Die Cyclopentadienylgruppen L' in Formel V können unsubstituiert oder substituiert sein. Sie sind gleich oder verschieden, bevorzugt gleich.

Beispiele für substituierte Cyclopentadienylgruppen L' sind: Tetramethylcyclopentadienyl, 3-Methylcyclopentadienyl, 3-tert.butylcyclopentadienyl, Methyl-tert.butylcyclopentadienyl, Isopropylcyclopentadienyl, Dimethylcyclopentadienyl, Trimethylcyclopentadienyl, Trimethylethylcyclopentadienyl, 3-Phenylcyclopentadienyl, Diphenylcyclopentadienyl, Indenyl, 2-Methylindenyl, 2-Ethylindenyl, 3-Methylindenyl, 3-Tert.butylindenyl, 3-Trimethylsilylindenyl, 2-Methyl-4-phenylindenyl, 2-Ethyl-4-phenylindenyl, 2-Methyl-4-naphthylindenyl, 2-Methyl-4-isopropylindenyl, Benzoindenyl, 2-Methyl-4,5-benzoindenyl, 2-Methyl-a-acenanaphthindenyl, 2-Methyl-4,6-diisopropylindenyl, Fluorenyl, 2-Methylfluorenyl oder 2,7-Di-tert.butylfluorenyl.

Eine oder beide Cyclopentadienylgruppen L' sind eine substituierte Cyclopentadienylgruppe, insbesondere ein Indenylderivat wie Indenyl, 2-Methylindenyl, 2-Ethylindenyl, 3-Methylindenyl, 3-Tert.butylindenyl, 3-Trimethylsilylindenyl, 2-Methyl-4-phenylindenyl, 2-Ethyl-4-phenylindenyl, 2-Methyl-4-naphthylindenyl, 2-Methyl-4-isopropylindenyl, Benzoindenyl, 2-Methyl-4,5-benzoindenyl, 2-Methyl-α-acenanaphthindenyl, 2-Methyl-4,6-diisopropylindenyl oder ein Fluorenylderivat wie Fluorenyl, 2-Methylfluorenyl oder 2,7-Di-tert.butylfluorenyl.

Die Reste R¹ und R² sind gleich oder verschieden, bevorzugt gleich, und bedeuten C₁-C₃₀-Kohlenwasserstoffreste wie C₁-C₁₀-Alkyl oder C₆-C₁₄-Aryl. Die Reste R¹ und R² können auch zusammen mit den sie verbindenen Atomen ein Ringsystem bilden, welches bevorzugt 4 bis 40, besonders bevorzugt 5 bis 15 Kohlenstoffatome enthält.

Bevorzugt ist M¹ ein Element der Gruppe IV des Periodensystems der Elemente wie Titan, Zirkonium oder Hafnium, insbesondere Zirkonium, R¹ und R² sind gleich oder verschieden, bevorzugt gleich sind, und bedeuten Wasserstoff, C₁-C₁₀-Alkyl oder C₆-C₁₄-Aryl, insbesondere C₁-C₅ Alkyl, und die Reste R¹¹ und R¹² sind bevorzugt gleich und bedeuten C₁-C₄-Alkyl wie Methyl oder ein Halogenatom wie Chlor.

Beispiele für nach dem erfindungsgemäßen Metallocen-Herstellungsverfahren erhältliche kohlenstoffverbrückte Biscyclopentadienyl-Metallocene sind: Isopropyliden-bis(2,3,4,5-tetramethylcyclopentadienyl)zirkoniumdichlorid, Methylnaphthylmethylen-bis(2,3,4-trimethylcyclopentadienyl)zirkoniumdichlorid, Diphenylmethylen-bis(2,3,4,5-tetrarnethylcyclopentadienyl)zirkoniumdimethyl, Methylen-bis(1-indenyl)zirkoniumdichlorid, lsopropyliden-bis(1-indenyl)zirkoniumdichlorid, Methylphenylmethylen-bis(1-indenyl)zirkoniumdichlorid, Diphenylmethylen-bis(1-indenyl)zirkoniumdichlorid, Methylen-bis(1-(4-phenylindenyl))zirkoniumdichlorid, lsopropyliden-bis(1-(4-phenylindenyl))zirkoniumdichlorid, Isopropyliden-bis(1-(4-naphtylindenyl))zirkoniumdichlorid, Methylphenylmethylen-bis(1-(4-phenylindenyl))zirkoniumdichlorid, Diphenylmethylen-bis(1-(4-phenylindenyl))zirkoniumdichlorid, Methylen-bis(1-(4-isopropylindenyl))zirkoniumdichlorid, Isopropyliden-bis(1-(4-isopropylindenyl))zirkoniumdichlorid, Methylphenylmethylen-bis(1-(4-isopropylindenyl))zirkoniumdimethyl, Diphenylmethylen-bis(1-(4-isopropylindenyl))hafniumdichlorid, Methylen-bis(1-(4,5-benzoindenyl))zirkoniumdichlorid, Isopropyliden-bis(1-(4,5-benzoindenyl)zirkoniumdichlorid, Methylphenylmethylen-bis(1-(4,5-benzoindenyl))zirkoniumdichlorid, Diphenylmethylen-bis(1-(4,5-benzoindenyl))zirkoniumdichlorid, Isopropyliden-(1-indenyl)-cyclopentadienyl-zirkoniumdichlorid, lsopropyliden-(1-indenyl)-(3-methylcyclopentadienyl)-zirkoniumdichlorid, Methylphenylmethylen-(1-indenyl)-cyclopentadienyl-zirkoniumdichlorid, Diphenylmethylen-(1-indenyl)-cyclopentadienyl-zirkoniumdichlorid, Diphenylmethylen-(1-(4-isopropyl)indenyl)-cyclopentadienyl-zirkoniumdichlorid, Isopropyliden-(1-indenyl)-cyclopentadienyl-titandichlorid, lsopropyliden-(1-indenyl)-3-methylcyclopentadienyl-titandichlorid, Methylphenylmethylen-(1-indenyl)-cyclopentadienyl-titandichlorid, Diphenylmethylen-(1-indenyl)-cyclopentadienyl-titandichlorid, Isopropyliden-(1-indenyl)-(9-fluorenyl)-zirkoniumdichlorid, Isopropyliden-(9-fluorenyl)-(3-methylcyclopentadienyl)-zirkoniumdichlorid, lsopropyliden-(9-fluorenyl)-(3-tert.butylcyclopentadienyl)-zirkoniumdichlorid, Methylphenylmethylen-(9-fluorenyl)-cyclopentadienyl-zirkoniumdichlorid, Diphenylmethylen-(9-fluorenyl)-cyclopentadienyl-zirkoniumdichlorid, Diphenylmethylen-(9-fluorenyl)-(3-phenylcyclopentadienyl)-zirkoniumdichlorid, Diphenylmethylen-(1-(4-isopropyl)indenyl)-(9-fluorenyl)-zirkoniumdichlorid, Isopropyliden-(9-fluorenyl)-cyclopentadienyl-zirkoniumdichlorid, Methylphenylmethylen-(9-fluorenyl)-cyclopentadienyl-titandichlorid, Diphenylmethylen-(9-fluorenyl)-cyclopentadienyl-titandimethyl, Diphenylmethylen-(9-(2,7-di-tert.butyl)fluorenyl)-cyclopentadienylzirkoniumdichlorid, Isopropyliden-(9-(2,7-di-tert.butyl)fluorenyl)-cyclopentadienyl-zirkoniumdichlorid.

Die vorliegende Erfindung betrifft somit auch ein Verfahren zur Herstellung eines kohlenstoffverbrückten Biscyclopentadienyl-Metallocens, enthaltend die Schritte:
a) Umsetzung einer oder zweier Cyclopentadienverbindungen LH wovon mindestens eine Cyclopentadienverbindung eine substituierte Cyclopentadienverbindung ist, mit einer Carbonylverbindung in Gegenwart mindestens einer Base und mindestens eines Phasentransferkatalysators wobei die Umsetzung a) in einem Zweiphasensystem durchgeführt wird, welches aus einem organischen Lösemittel und Wasser besteht, zu einer kohlenstoffverbrückten Biscyclopentadienverbindung, und
b) Umsetzung der in Schritt a) erhaltenen kohlenstoffverbrückten Biscyclopentadienverbindung mit einer Metallverbindung M¹Xₚ, worin M¹ ein Element der Gruppe IIIb, IVb, Vb oder VIb des Periodensystems der Elemente ist, X ein C₁-C₄₀-kohlenstoffhaltiger Rest, wie C₁-C₁₀Alkyl oder NR¹³₂, worin R¹³ ein C₁-C₂₀-Kohlenwasserstoffrest wie C₁-C₁₀-Alkyl oder C₆-C₁₆-Aryl ist, ein Halogen oder ein Pseudohalogen ist und p eine ganze Zahl von 0 bis 4 ist, unter Bedingungen, unter denen die in Schritt a) erhaltene kohlenstoffverbrückte Biscyclopentadienverbindung zum kohlenstoffverbrückten Biscyclopentadienyl-Metallocen komplexiert wird.

Der zweite Schritt (b) des Herstellungsverfahrens des kohlenstoffverbrückten Biscyclopentadienyl-Metallocens kann nach literaturbekannten Verfahren durchgeführt werden (z.B. AU-A-31478/89; J. Organomet. Chem. 1988, 342, 21 oder EP-A 284 707 auf die hiermit ausdrücklich Bezug genommen wird). Bevorzugt wird die kohlenstoffverbrückte Biscyclopentadienverbindung zunächst mit einer Verbindung der Formel R¹⁴M² umgesetzt, worin M² ein Metall der Gruppe la, lla oder IIIa des Periodensystems der Elemente bedeutet und R¹⁴ ein C₁-C₂₀-Kohlenwasserstoffrest wie C₁-C₁₀-Alkyl oder C₆-C₁₄-Aryl ist, und anschließend mit der Metallverbindung M¹Xₚ umgesetzt. Die Umsetzungen finden bevorzugt in einem geeigneten Lösemittel z.B. einem aliphatischen oder aromatischen Lösemittel, wie Hexan oder Toluol, einem etherischen Lösemittel, wie Tetrahydrofuran oder Diethylether oder in halogenierten Kohlenwasserstoffen, wie Methylenchlorid oder o-Dichlorbenzol statt. In der Metallverbindung der Formel M¹Xₚ ist M¹ bevorzugt ein Element der Gruppe IIIb des Periodensystems der Elemente, X ist bevorzugt ein Halogenatom oder NR¹³₂, worin R¹³ ein C₁-C₁₀-Kohlenwasserstoffrest wie C₁-C₁₀-Alkyl oder C₆-C₁₀-Aryl ist und p ist bevorzugt 4. Die kohlenstoffverbrückte Biscyclopentadienylverbindung kann als Isomerengemisch eingesetzt werden.

Kohlenstoffverbrückte Biscyclopentadienyl-Metallocenhalogenide der Formel V können nach literaturbekannten Methoden, z.B. durch Umsetzung mit Alkylierungsmitteln, wie z.B. Lithiumalkylen, zu den entsprechenden Mono- oder Dialkylverbindungen derivatisiert werden (J.Am. Chem. Soc. 1973, 95, 6263).

Die kohlenstoffverbrückten Biscyclopentadienyl-Metallocene der Formel V können als Gemisch der racemischen Form und der meso-Form anfallen. Die Trennung der isomeren Formen, insbesondere die Abtrennung der meso-Form, ist im Prinzip bekannt (AU-A-31478/89; J. Organomet. Chem. 1988, 342, 21; EP-A 284 707) und kann durch Extraktion oder Umkristallisation mit verschiedenen Lösemitteln erfolgen.

Das erfindungsgemäße Verfahren erlaubt mit hoher Ausbeute die einfache Herstellung von kohlenstoffverbrückten Biscyclopentadienyl-Metallocenen.

Die mit dem erfindungsgemäßen Metallocen-Herstellungsverfahren erhältlichen kohlenstoffverbrückten Biscyclopentadienyl-Metallocene können zusammen mit einem Cokatalysator als hochaktive Katalysatorkomponenten z.B. für die Herstellung von Olefinpolymeren eingesetzt werden.

Polymerisiert werden können Olefine, insbesondere solche der Formel R^{a}-CH=CH-R^{b}, worin R^{a} und R^{b} gleich oder verschieden sind und ein Wasserstoffatom oder einen Kohlenwasserstoffrest mit **1** bis **20** Kohlenstoffatomen bedeuten. R^{a} und R^{b} können auch mit den sie verbindenen Kohlenstoffatomen einen Ring bilden. Beispiele für solche Olefine sind Ethylen, Propylen, 1-Buten, 1-Hexen, 1-Octen, 4-Methyl-1-penten, 1,3-Butadien, Isopren, Norbornen, Dimethanooctahydronaphthalin oder Norbornadien. Insbesondere können Propylen und Ethylen homopolymerisiert werden, Ethylen mit einem C₃-C₂₀-Olefin und/oder einem C₄-C₂₀-Dien copolymerisiert werden oder Ethylen mit einem Cycloolefin copolymerisiert werden.

Die Polymerisation kann eine Homo- oder eine Copolymerisation sein und in Lösung, in Suspension oder in der Gasphase, kontinuierlich oder diskontinuierlich, ein- oder mehrstufig bei einer Temperatur von 0 bis 200°C, vorzugsweise 30 bis 100°C durchgeführt werden.

Prinzipiell ist als Cokatalysator in der Polymerisation jede Verbindung geeignet, die aufgrund ihrer Lewis-Acidität das neutrale Metallocen in ein Kation überführen und dieses stabilisieren kann ("labile Koordination). Darüber hinaus soll der Cokatalysator oder das aus ihm gebildete Anion keine weiteren Reaktionen mit dem gebildeten Kation eingehen (EP 427 697). Als Cokatalysator wird bevorzugt eine Aluminiumverbindung und/oder Borverbindung eingesetzt.

Als Cokatalysatoren werden bevorzugt Aluminoxane verwendet (EP-A 129-368), Polyhedron 1990. 9, 429). An Stelle oder neben eines Aluminoxans können Borverbindungen, insbesondere der Formeln RₓNH_{4-X}BR₄', RₓPH₄₋ₓBR₄', R₃CBR₄' oder BR₃' als Cokatalysatoren verwendet werden. In diesen Formeln bedeutet x eine ganze Zahl von 1 bis 4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit den sie verbindenen Atomen einen Ring, und die Reste R'sind gleich oder verschieden, bevorzugt gleich, und stehen für C₆-C₁₈-Alkyl oder C₆-C₁₈-Aryl, das durch Alkyl, Haloalkyl oder Fluor substituiert sein kann (EP-A 277 003, 277 004, 426 638, 427697).

Es ist möglich, das Metallocen vor dem Einsatz in der Polymerisationsreaktion mit einem Cokatalysator, insbesondere einem Aluminoxan vorzuaktivieren. Dadurch kann die Polymerisationsaktivität deutlich erhöht werden. Die Voraktivierung des Metallocens wird vorzugsweise in Lösung vorgenommen. Bevorzugt wird dabei das Metallocen in eine Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Zur Entfernung von im Olefin vorhandenen Katalysatorgiften ist eine Reinigung mit einer Aluminiumverbindung, bevorzugt einem Aluminiumalkyl, wie Trimethylaluminium oder Triethylaluminium, vorteilhaft. Diese Reinigung kann sowohl im Polymerisationssystem selbst erfolgen, oder das Olefin wird vor der Zugabe in das Polymerisationssystem mit der Aluminiumverbindung in Kontakt gebracht und anschließend wieder abgetrennt.

Als Molmassenregler und/oder zur Steigerung der Katalysatoraktivität kann in dem Polymerisationsverfahren Wasserstoff zugegeben werden. Hierdurch können niedermolekulare Polyolefine wie Wachse erhalten werden.

Bevorzugt wird das Metallocen mit dem Cokatalysator außerhalb des Polymerisationsreaktors in einem separaten Schritt unter Verwendung eines geeigneten Lösemittels umgesetzt. Dabei kann eine Trägerung vorgenommen werden.

In dem Verfahren kann mit Hilfe des Metallocens eine Vorpolymerisation erfolgen. Zur Vorpolymerisation wird bevorzugt das (oder eines der) in der Polymerisation eignesetze(n) Olefin(e) verwendet.

Der zur Olefinpolymerisation eingesetzte Katalysator kann geträgert sein. Durch die Trägerung läßt sich beispielsweise die Kornmorphologie des hergestellten Polymers steuern. Dabei kann das Metallocen zunächst mit dem Träger und anschließend mit dem Cokatalysator umgesetzt werden. Es kann auch zunächst der Cokatalysator geträgert werden und anschließend mit dem Metallocen umgesetzt werden. Auch ist es möglich das Reaktionsprodukt von Metallocen und Cokatalysator zu trägern. Geeignete Trägermaterialien sind beispielsweise Silikagele, Aluminiumoxide, festes Aluminoxan oder andere anorganische Trägermaterialien wie beispielsweise Magnesiumchlorid. Ein geeignetes Trägermaterial ist auch ein Polyolefinpulver in feinverteilter Form. Die Herstellung des geträgerten Cokatalysators kann beispielsweise wie in EP 567 952 beschrieben durchgeführt werden.

Vorzugsweise wird der Cokatalysator , z.B. Aluminoxan, auf einen Träger wie beispielsweise Silikagele, Aluminiumoxide, festes Aluminoxan oder andere anorganische Trägermaterialien wie beispielsweise Magnesiumchlorid oder auch ein Polyolefinpulver in feinverteilter Form aufgebracht und dann mit dem Metallocen umgesetzt.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Hexan, Heptan, Isooctan, Cyclohexan, Methylcyclohexan genannt. Weiterhin kann auch eine Benzin- bzw. hydrierte Dieselölfraktion benutzt werden. Brauchbar ist auch Toluol. Bevorzugt wird im flüssigen Monomeren polymerisiert.

Durch Anwendung von Wasserstoff oder durch Erhöhung der Polymerisationstemperatur sind auch Polyolefine niedriger Molmasse, wie Wachse zugänglich, deren Härte oder Schmelzpunkt durch den Comonomergehalt variiert werden können. Durch Wahl des Polymerisations-Verfahrens und der Comonomerart(en), sowie Comonomermenge(n) lassen sich Olefincopolymere mit elastomeren Eigenschaften, wie z.B. Ethylen/Propylen/1,4-Hexadien-Terpolymere herstellen.

Die nachfolgenden Beispiele dienen zur Erläuterung der Erfindung.

### 1) 2,2-Bisindenylpropan

100,0 g (0,86 mol) Inden werden in 400 ml Toluol gelöst und anschließend wird eine Lösung von 86,2 g (2,2 mol) Natriumhydroxid und 19,6 g (86 mmol) Triethylbenzylammoniumchlorid in 86,2 ml Wasser zugegeben (50%-iger NaOH-Lösung). Die Zugabe von 25,0 g (0,43 mol) Aceton erfolgt tropfenweise über einen Zeitraum von 30 min. Nach einer Reaktionszeit von 5 Stunden wird die wäßrige Phase abgetrennt, zweimal mit jeweils 100 ml Diethylether extrahiert und die vereinigten organischen Phasen über MgSO₄ getrocknet. Das Lösungsmittel wird im Vakuum entfernt und das Rohprodukt durch Umkristallisation aus Toluol/Hexan gereinigt. Man erhält 99,6 g 2,2-Bisindenylpropan in 85%-iger Ausbeute in Form eines gelben Pulvers.
¹H-NMR (200 MHz, CDCl₃): 7,4 - 6,9 (m, 8H, arom. H), 6,42 (s, 2H, Olefin, H), 3,35 (s, 4H, CH₂), 1,70 (s, 6H, CH₃). Massenspektrum: 272 M+, korrektes Zerfallsmuster.

### 2) 1,1-Bisindenylethan

100,0 g (0,86 mol) Inden werden in 400 ml Toluol gelöst und anschließend wird eine Lösung von 86,2 g (2,2 mol) Natriumhydroxid und 19,6 g (86 mmol) Triethylbenzylammoniumchlorid in 86,2 ml Wasser zugegeben (50%-iger NaOH-Lösung). Die Zugabe von 18,9 g (0,43 mol) Acetaldehyd erfolgt tropfenweise über einen Zeitraum von 30 min. Nach einer Reaktionszeit von 5 Stunden wird die wäßrige Phase abgetrennt, zweimal mit jeweils 100 ml Diethylether extrahiert und die vereinigten organischen Phasen über MgSO₄ getrocknet. Das Lösungsmittel wird im Vakuum entfernt und das Rohprodukt durch Umkristallisation aus Toluol/Hexan gereinigt. Man erhält 91,5 g 1,1-Bisindenylethan in 82%-iger Ausbeute in Form eines gelben Pulvers.
¹H-NMR (200 MHz, CDCl₃): 7,3 - 6,9 (m, 8H, arom. H), 6,47 (s, 2H, Olefin, H), 3,41 (s, 4H, CH₂), 3,10 (s, 1H, CH), 1,65 (s, 3H, CH₃). Massenspektrum: 259 M+, korrektes Zerfallsmuster.

### 3) lsopropyliden-(bis-1-indenyl)-zirkoniumdichlorid

Eine Lösung von 10 g (37 mmol) 2,2-Bisindenylpropan wird in 30 ml Diethylether bei Raumtemperatur unter Argonschutz mit 29,6 ml (74 mmol) einer 2.5 M Butyllithiumlösung in Hexan versetzt und über Nacht gerührt. Nach Zusatz von 20 ml Hexan wird die beigefarbene Suspension filtriert und der Rückstand mit 20 ml Pentan gewaschen. Das Dilithiosalz wird im Ölpüumpenvakuum getrocknet und dann bei -78°C zu einer Suspension von 8,6 g (37 mmol) ZrCl4 in Dichlormethan gegeben. Die Mischung wird innerhalb 1 h auf Raumtemperatur erwärmt und noch 30 min bei dieser Temperatur gerührt. Nach dem Abziehen des Lösemittels wird der orangebraune Rückstand mit 50 ml Toluol extrahiert. Nach Abziehen des Lösemittels erhält man 8,8 g (55%) eines orangen Pulvers. Das Verhältnis des Racemats zur meso-Form wurde zu 2 1 bestimmt. Durch Umkristallisation aus Toluol konnten 4,1 g (26%) des reinen Racemats gewonnen werden.
¹H-NMR (200 MHz, CDCl₃): 7,8 - 6.9 (m, 8H, arom. H), 6,72 (m, 2H, Cp-H), 6,17 (m, 2H, Cp-H), 2,15 (s, 6H, CH₃). Massenspektrum: 432 M+, korrektes Zerfallsmuster.

## Patentansprüche

1. Verfahren zur Herstellung einer kohlenstoffverbrückten Biscyclopentadienverbindung durch Umsetzung einer oder zweier Cyclopentadienverbindungen LH, wovon mindestens eine Cyclopentadienverbindung eine substituierte Cyclopentadienverbindung ist, mit einer Carbonylverbindung in Gegenwart mindestens einer Base und mindestens eines Phasentransferkatalysators, wobei das Verfahren in einem Zweiphasensystem durchgeführt wird, welches aus einem organischen Lösemittel und Wasser besteht.

2. Verfahren gemäß Anspruch 1, worin die Base ein Hydroxid eines Elements der Gruppe la, IIa oder IIIa des Periodensystems der Elemente ist.

3. Verfahren gemäß einem oder mehreren der Ansprüche 1 bis 2, worin der Phasentransferkatalysator ein quartäres Ammoniumsalz, ein quartäres Phosphoniumsalz oder eine Kronenverbindung ist.

4. Verfahren gemäß einem oder mehreren der Ansprüche 1 bis 3, worin die kohlenstoffverbrückte Biscyclopentadienverbindung die Formel I aufweist 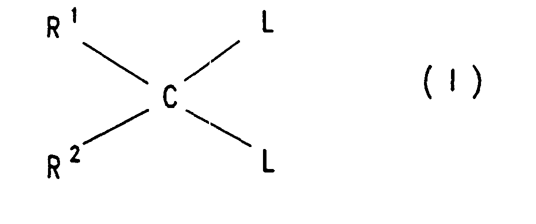 , worin L unabhängig voneinander gleich oder verschieden eine Cyclopentadiengruppe sind, wobei mindestens eine Gruppe L eine substituierte Cyclopentadienylgruppe ist, und R¹ und R² gleich oder verschieden sind und ein Wasserstoffatom oder einen C₁-C₃₀-Kohlenwasserstoffrest bedeuten.

5. Verfahren gemäß einem oder mehreren der Ansprüche 1 bis 4, worin in Formel I beide Cyclopentadiengruppen L substituierte Cyclopentadiengruppen sind.

6. Verfahren zur Herstellung eines kohlenstoffverbrückten Biscyclopentadienyl-Metallocens, enthaltend die Schritte:
a) Herstellung einer kohlenstoffverbrückten Biscyclopentadienverbindung nach dem Verfahren gemäß einem oder mehreren der Ansprüche 1 bis 5, und
b) Umsetzung der in Schritt a) erhaltenen kohlenstoffverbrückten Biscyclopentadienverbindung mit einer Metallverbindung M¹Xₚ, worin M¹ ein Element der Gruppe IIIb, IVb, Vb oder Vlb des Periodensystems der Elemente ist, X ein C₁-C₄₀-kohlenstoffhaltiger Rest, ein Halogen oder ein Pseudohalogen ist und p eine ganze Zahl von 0 bis 4 ist, unter Bedingungen, unter denen die in Schritt a) erhaltene kohlenstoffverbrückte Biscyclopentadienverbindung zum kohlenstoffverbrückten Biscyclopentadienyl-Metallocen komplexiert wird.

7. Verwendung des Verfahrens gemäß einem oder mehreren der Ansprüche 1 bis 5 als Teilschritt eines Verfahrens zur Herstellung eines kohlenstoffverbrückten Biscyclopentadienyl-Metallocens.

8. Verwendung eines Phasentransferkatalysators in einem Verfahren zur Herstellung einer kohlenstoffverbrückten Biscyclopentadienverbindung durch Umsetzung einer oder zweier Cyclopentadienverbindungen LH, wovon mindestens eine Cyclopentadienverbindung eine substituierte Cyclopentadienverbindung ist, mit einer Carbonylverbindung in Gegenwart eines Hydroxyds eines Elements der Gruppe Ia, IIa oder IIIa des Periodensystems der Elemente, in einem Zweiphasensystem, welches aus einem organischen Lösemittel und Wasser besteht.

## Claims

1. A process for preparing a carbon-bridged biscyclopentadiene compound by reacting one or two cyclopentadiene compounds LH, of which at least one cyclopentadiene compound is a substituted cyclopentadiene compound, with a carbonyl compound in the presence of at least one base and at least one phase transfer catalyst, where the process is carried out in a two-phase system comprising an organic solvent and water.

2. A process as claimed in claim 1, wherein the base is a hydroxide of an element of group Ia, IIa or IIIa of the Periodic Table of the Elements.

3. A process as claimed in claim 1 or 2, wherein the phase transfer catalyst is a quaternary ammonium salt, a quaternary phosphonium salt or a crown compound.

4. A process as claimed in one or more of claims 1 to 3, wherein the carbon-bridged biscyclopentadiene compound has the formula I 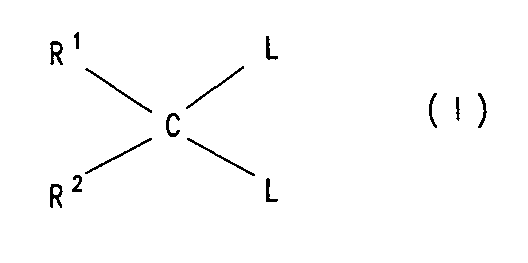 where L are, independently of one another, identical or different cyclopentadiene groups, where at least one group L is a substituted cyclopentadienyl group, and R¹ and R² are identical or different and are each a hydrogen atom or a C₁-C₃₀-hydrocarbon radical.

5. A process as claimed in one or more of claims 1 to 4, wherein, in formula I, both cyclopentadiene groups L are substituted cyclopentadiene groups.

6. A process for preparing a carbon-bridged biscyclopentadienyl metallocene, comprising the steps:
a) preparing a carbon-bridged biscyclopentadiene compound by the process as claimed in one or more of claims 1 to 5, and
b) reacting the carbon-bridged biscyclopentadiene compound obtained in step a) with a metal compound M¹Xₚ, where M¹ is an element of group IIIb, IVb, Vb or VIb of the Periodic Table of the Elements, X is a C₁-C₄₀-radical, a halogen or a pseudohalogen and p is an integer from 0 to 4, under conditions under which the carbon-bridged biscyclopentadiene compound obtained in step a) is complexed to give the carbon-bridged biscyclopentadienyl metallocene.

7. The use of the process as claimed in one or more of claims 1 to 5 as a substep of a process for preparing a carbon-bridged biscyclopentadienyl metallocene.

8. The use of a phase transfer catalyst in a process for preparing a carbon-bridged biscyclopentadiene compound by reacting one or two cyclopentadiene compounds LH, of which at least one cyclopentadiene compound is a substituted cyclopentadiene compound, with a carbonyl compound in the presence of a hydroxide of an element of group Ia, IIa or IIIa of the Periodic Table of the Elements in a two-phase system comprising an organic solvent and water.

## Revendications

1. Procédé de préparation d'un composé bis-cyclopentadiène ponté par des atomes de carbone, dans lequel on met à réagir un ou deux composés cyclopentadiène LH, dont au moins un composé cyclopentadiène est un composé cyclopentadiène substitué, et un composé carbonyle en présence d'au moins une base et d'au moins un catalyseur de transfert de phase, et on réalise la réaction dans un système à deux phases se composant d'un solvant organique et d'eau.

2. Procédé selon la revendication 1, dans lequel la base est un hydroxyde d'un élément des Groupes Ia, IIa ou IIIa de la Classification Périodique des Eléments.

3. Procédé selon une ou plusieurs des revendications 1 à 2, dans lequel le catalyseur de transfert de phase est un sel d'ammonium quaternaire, un sel de phosphonium quaternaire ou un composé couronne.

4. Procédé selon une ou plusieurs des revendications 1 à 3, dans lequel le composé bis-cyclopentadiène ponté par des atomes de carbone possède la formule I 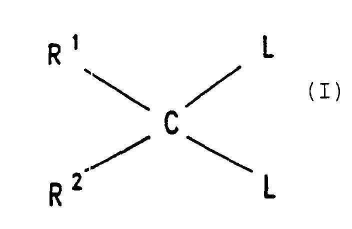 dans laquelle L, indépendamment l'un de l'autre, identiques ou différents, représente un groupe cyclopentadiène, avec au moins un des groupes L représentant un groupe cyclopentadiène substitué, et R¹ et R² sont identiques ou différents et représentent un atome d'hydrogène ou un résidu hydrocarbure en C₁ à C_{30.}

5. Procédé selon une ou plusieurs des revendications 1 à 4, dans lequel, dans la formule I, les deux groupes cyclopentadiène L sont des groupes cyclopentadiène substitués.

6. Procédé de préparation d'un métallocène-bis-cyclopentadiényle ponté par des atomes de carbone, comprenant les étapes suivantes consistant :
a) à préparer un composé bis-cyclopentadiène ponté par des atomes de carbone à l'aide du procédé selon une ou plusieurs des revendications 1 à 5 et
b) à faire réagir le composé bis-cyclopentadiène ponté par des atomes de carbone obtenu dans l'étape a) avec un composé métallique M^{1X}ₚ, dans laquelle M¹ représente un élément des Groupes IIIb, IVb, Vb ou VIb de la Classification Périodique des Eléments, X représente un résidu hydrocarbure en C₁ à C₄₀, un atome d'halogène ou de pseudohalogène et p est un nombre entier de 0 à 4, dans des conditions dans lesquelles est réalisé la complexation du composé bis-cyclopentadiène ponté par des atomes de carbone obtenu dans l'étape a) en métallocène-bis-cyclopentadiényle ponté par des atomes de carbone.

7. Utilisation du procédé selon une ou plusieurs des revendications 1 à 5 en tant qu'étape partielle d'un procédé de fabrication d'un métallocène-bis-cyclopentadiényle ponté par des atomes de carbone.

8. Utilisation d'un catalyseur de transfert de phase dans le procédé de fabrication d'un composé bis-cyclopentadiène ponté par des atomes de carbone consistant à faire réagir un ou deux composés cyclopentadiène LH, dont au moins un composé cyclopentadiène est un composé cyclopentadiène substitué, avec un composé carbonyle, en présence de l'hydroxyde d'un élément des Groupes Ia, IIa ou IIIa de la Classification Périodique des Eléments, dans un système à deux phases se composant d'un solvant organique et de l'eau.
